# EUROPÄISCHE PATENTANMELDUNG

(11) **EP 0 757 057 A2**
(43) Veröffentlichungstag der Anmeldung: **05.02.1997**
(21) Anmeldenummer: 96110784.4
(22) Anmeldetag: 04.07.1996
(51) Int. Cl.: C07K 1/30, C07H 21/00

(54) **Verfahren zur Kristallisierung von Makromolekülen**

(30) Priorität: 03.08.1995 DE 19528507
(71) Anmelder: BEHRINGWERKE Aktiengesellschaft, 35001 Marburg (DE)
(72) Erfinder: Fibi, Mathias, Dr., 35041 Marburg (DE)

(57) **Zusammenfassung**

Die Erfindung betrifft ein Verfahren zur Kristallisierung von Makromolekülen, wobei die Kristallisierung in Gegenwart von 4-Alkyl-Umbelliferon-Salzen erfolgt.

## Beschreibung

Die vorliegende Erfindung betrifft ein Verfahren zur Kristallisierung von Makromolekülen, sowie die mit diesen Verfahren herstellbaren Makromoleküle in kristalliner Form.

Die kristalline Darstellung von Makromolekülen ist allgemein von großem Interesse. So wird beispielsweise die Röntgenstrukturanalyse von Polypeptid- und Proteinkristallen neben der Elektrophorese, der Peptid-Kartierung und der Sequenzierung zur Charakterisierung dieser Moleküle herangezogen. Durch die Röntgenstrukturanalyse ist es außerdem möglich, die dreidimensionale Struktur eines Makromoleküls zu bestimmen. Dies wiederum ist die Voraussetzung für "computer-assisted molecular-design" (CAMD) von Ligandenagonisten, Rezeptoragonisten, Rezeptorantagonisten, Enzymsubstraten, Enzyminhibitoren, Bindungsstellenagonisten, Bindungsstellenantagonisten, etc.. Mit Hilfe der Röntgenstrukturanalyse können außerdem prinzipielle Struktur-Funktions-Beziehungen aufgeklärt werden.

Darüberhinaus besitzen kristallisierte Makromoleküle im allgemeinen eine höhere Lagerstabilität, so daß beispielsweise bei pharmazeutischen Darreichungsformen auch bei längerer Lagerung kein Aktivitätsverlust auftritt.

Es wird daher angestrebt, Makromoleküle oder deren Derivate in kristalliner Form darzustellen, bevorzugt mit einer Kristallqualität, die eine Röntgenstrukturanalyse zuläßt.

Es hat sich jedoch gezeigt, daß Makromoleküle, insbesondere modifizierte Proteine wie beispielsweise Glycoproteine, meist nur schwierig zu kristallisieren sind. Außerdem ist die Güte der Kristalle häufig nicht ausreichend, um gesicherte röntgenkristallographische Daten zu erhalten.

Außerdem können kleine Kristalle als Saat- oder Impfrkistalle für die Kristallzüchtung verwendet werden, die dann zum Entstehen von größeren Kristallen führen. Dieses Verfahren setzt allerdings voraus, daß zumindest überhaupt Kristalle des gewünschten Makromoleküls erhalten werden können.

Im Stand der Technik sind verschiedene Verfahren bekannt, um Makromoleküle zu kristallisieren. Dabei müssen die Makromoleküle einen bestimmten, meist sehr hohen Reinheitsgrad und eine relativ hohe Homogenität besitzen. Bekannt ist beispielsweise die Kristallisierung von Makromolekülen in Gegenwart von Polyethylenglycol oder verschiedenen Salzen.

So beschreiben D.S. Katz et al. in Biochemical and Biophysical Research Communications 1993, 196, S. 752-757 die Kristallisierung von Antichymotrypsin-Varianten nach der "sitting drop"-Methode in Gegenwart eines Puffers enthaltend Polyethylenglycol, 2-[N-Morpholino]ethylsulfonsäure-NH₄OH und CaCl₂ bzw. Ammoniumsulfat und Natriumcitrat.

Die Kristallisierung eines DNA-Anthramycin-Adducts in Gegenwart von MgCl₂ und 2-Methyl-2,4-pentandiol aus Methanol wird von M.L. Kopka et al. in Biochemistry 1994, 33, S. 13593-13610 beschrieben.

Nachteilig bei den vorbekannten Verfahren ist jedoch, daß diese Verfahren relativ spezifisch für die jeweiligen Makromoleküle sind.

Es ist somit eine Aufgabe der vorliegenden Erfindung, ein neues Verfahren zur Kristallisierung von Makromolekülen zur Verfügung zu stellen.

Es wurde überraschend gefunden, daß Makromoleküle in Gegenwart von 4-Alkyl-Umbelliferon-Salzen besonders leicht kristallisieren.

Die vorliegende Erfindung betrifft daher ein Verfahren zur Kristallisierung von Makromolekülen, das dadurch gekennzeichnet ist, daß die Kristallisierung in Gegenwart eines oder mehrerer 4-Alkyl-Umbelliferon-Salze erfolgt, wobei die Alkylgruppe geradkettig oder verzweigt sein kann und 1 bis 18 Kohlenstoffatome aufweist.

Umbelliferon ist ein 7-Hydroxycumarin-Derivat, so daß 4-Alkyl-Umbelliferon ein 4-Alkyl-7-hydroxy-2H-benzo-1-pyran-2-on darstellt. Im Rahmen der vorliegenden Erfindung werden bevorzugt solche 4-Alkyl-Umbelliferon-Salze eingesetzt, bei denen die Alkylgruppe 1 bis 8, und besonders bevorzugt 1 bis 4 Kohlenstoffatome aufweist.

Als Salze der 4-Alkyl-Umbelliferone eignen sich insbesondere die Alkali- und/oder Erdalkalisalze, wobei die Natrium- und/oder Kaliumsalze bevorzugt sind.

Besonders bevorzugt ist das 4-Alkyl-Umbelliferon-Salz ein 4-Methyl-Umbelliferon-Salz, wobei das Natriumsalz ganz besonders bevorzugt ist.

Als Lösungsmittel eignen sich beispielsweise Wasser, Aceton und/oder Alkohole wie beispielsweise Ethylalkohol oder n- oder iso-Propylalkohol. Vorzugsweise erfolgt die Kristallisierung aus wäßriger Lösung. Die Lösung kann dabei weitere übliche Zusatzstoffe wie puffernde Verbindungen, Salze oder Schwermetallverbindungen zur Markierung der Makromoleküle für eine anschließende Röntgenstrukturanalyse umfassen.

Das erfindungsgemäße Verfahren ist prinzipiell nicht auf die Kristallisierung von bestimmten Makromolekülen beschränkt. Insbesondere eignet es sich zur Kristallisierung von Polypeptiden, Proteinen und Nukleinsäuren, wie DNA, sowie deren Derivate. Bevorzugt können Glycoproteine und Nucleinsäuren mit dem erfindungsgemäßen Verfahren kristallisiert werden.

Die Makromoleküle sollten zur Kristallisierung eine bestimmte Reinheit und Homogenität aufweisen. Als Polypeptide eignen sich beispielsweise solche, die durch Expression in E.coli, Hefe oder Animalzellen mit anschließender konventioneller Isolierung erhalten wurden oder synthetische Polypeptide. Die Reinigung kann über Affinitätschromatographie, konventionelle Chromatographie, rpHPLC, FPLC, usw. erfolgen. Nukleinsäuren können über eine konventionelle Isolierung beispielsweise nach deren Biosynthese als Plasmid oder einer sonstigen Synthese und konventioneller Reinigung erhalten werden.

Die Kristallisierungsbedingungen hängen von den zu kristallisierenden Makromolekülen, dem eingesetzten 4-Alkyl-Umbelliferon-Salz und dem Lösungsmittel sowie weiteren Faktoren ab. Die Bestimmung der jeweiligen optimalen Bedingungen gehört zur Laborroutine und kann vom Fachmann leicht ausgeführt werden.

Beispielsweise kann eine Lösung des Makromoleküls mit einer Konzentration von 0,1 bis 10.000 µg/ml im Verhältnis 1000:1 bis 1:1000 mit einer 0,1 bis 1000 mM 4-Methyl-Umbelliferon-Natriumsalz-Kristallisierungslösung gemischt und bei 0°C bis 25°C inkubiert werden. Vorzugsweise erfolgt die Inkubation bei 0°C bis 10°C und besonders bevorzugt bei etwa 4°C.

Die Makromoleküllösung kann beispielsweise eine Proteinlösung in, einem Phosphatpuffer bei einem pH von 3,0 bis 9,0, vorzugsweise bei einem pH von etwa 7,0 sein. Die Makromoleküllösung kann zusätzlich 1-500 mM 4-Methyl-Umbelliferon-Natriumsalz enthalten, wobei die 4-Methyl-Umbelliferon-Natriumsalz-Konzentration in dieser Lösung geringer ist als die 4-Methyl-Umbelliferon-Natriumsalz-Konzentration in der Kristallisierungslösung. Das Mischen der Makromoleküllösung und der Kristallisierungslösung kann beispielsweise auf einer Mikrotiterplatte erfolgen.

Nach dem erfindungsgemäßen Verfahren bilden sich Kristalle innerhalb weniger Stunden bis hin zu einigen Monaten.

Die 4-Alkyl-Umbelliferon-Salze dienen im erfindungsgemäßen Verfahren als Kristallisierungshilfsmittel, das in Gegenwart der zu kristallisierenden Makromoleküle kristallisiert, so daß die Makromoleküle in das entstehende Kristallgitter eingelagert werden.

Die durch das erfindungsgemäße Verfahren herstellbaren kristallisierten Makromoleküle weisen daher das 4-Alkyl-Umbelliferon-Salz und bevorzugt das 4-Methyl-Umbelliferon-Salz in der Kristallstruktur auf.

Die erfindungsgemäß kristallisierten Makromoleküle können in pharmazeutischen Zusammensetzungen als präventive oder therapeutische Darreichungsform verwendet werden, wobei durch die Kristallform der Makromoleküle eine besonders günstige Dosierung möglich ist. Beispielsweise sind die Kristalle zu peroralen, subkutanen, intrakutanen, peritonealen, intravenösen, intramuskulären usw. Verabreichungen geeignet. Die vorliegende Erfindung umfaßt somit ebenfalls pharmazeutische Zusammensetzungen, die als Wirkstoff eine pharmakologisch wirksame Menge eines erfindungsgemäß kristallisierten Makromoleküls und gegebenenfalls einen oder mehrere herkömmliche, pharmazeutisch akzeptable Träger umfassen.

Die erfindungsgemäß kristallisierten Makromoleküle können im Prinzip in gleicher Weise eingesetzt werden, wie es für viele Makromoleküle in pharmazeutischen Präparationen bekannt ist, beispielsweise als Depot-Präparation, um die tägliche Dosis von 0,001 µg/kg bis 100 mg/kg Körpergewicht des pharmakologisch wirksamen Makromoleküls zu verabreichen. Demnach können die erfindungsgemäß kristallisierten Formen der verschiedensten Makromoleküle beispielsweise als Therapeutikum-Depot, Antigen-Depot, DNA-Depot oder Zucker-Depot eingesetzt werden. Der im Kristall enthaltene Kristallisierungshilfsstoff wird dabei als Adjuvans (bei der Vaccinierung) verwendet.

4-Alkyl-Umbelliferon-Salze haben zudem den Vorteil, daß sie bereits alleine entzündungshemmend wirken und somit ebenfalls therapeutisch wirksam sind.

Die erfindungsgemäß kristallisierten DNA-Kristalle finden Anwendungsmöglickeiten sowohl in der Therapie mit gentechnologisch hergestellten Produkten als auch bei der Vaccinierung mit DNA.

Die erfindungsgemäß hergestellten Makromolekülkristalle können für verschiedene Analysen beispielsweise nach folgender Methode wieder aufgelöst werden:

Kristalle werden aus einem Mikrowell entnommen und dann mit Waschlösung bestehend aus 4-Alkyl-Umbelliferon-Salz in Puffer (derselbe Puffer wie bei der Kristallisierung verwendet) bei 4°C gewaschen. Die Suspension wird zentrifugiert und die Waschlösung wird entfernt. Die gewaschenen Kristalle werden anschließend in 100 µl 20 mM Natriumphosphat, pH 7,5 und 0,1 M NaCl bei 25°C gelöst. Bei Bedarf können die Kristalle gemörsert und anschließend gelöst werden.

In erfindungsgemäß hergestellten Makromolekülkristallen wurden die Makromoleküle beispielsweise wie folgt nachgewiesen:

### Nachweis von Protein in den Kristallen

Um Protein in den Kristallen nachzuweisen, werden Kristalle aus einem Mikrowell entnommen, fünfmal mit PBS gewaschen und anschließend in Protein-SDS-PAGE-Probenpuffer bei 95°C aufgelöst. Das Kristall-Lysat wird mit Hilfe der SDS-Polyacrylamidgelelektrophorese (SDS-PAGE) aufgetrennt und das Protein wird im Gel durch Silberfärbung nachgewiesen.

### Nachweis von Nukleinsäuren in den Kristallen

Um Nukleinsäuren in den Kristallen nachzuweisen, werden Kristalle aus einem Mikrowell entnommen, fünfmal mit PBS gewaschen und anschließend in DNS-Harnstoff-Probenpuffer aufgelöst. Das Kristall-Lysat wird in Harnstoff-Agarosegelen oder Harnstoff-PAGE aufgetrennt und die Nukleinsäuren werden im Gel durch Silberfärbung oder interkalierende Fluoreszenz-Farbstoffe (bsp. Ethidiumbromid) nachgewiesen.

Die folgenden Beispiele sollen die vorliegende Erfindung näher erläutern:

### Beispiel 1

### Kristallisierung von Makromolekülen

Eine Makromoleküllösung, die zwischen 0.1 und 10000 µg Makromolekül pro ml Lösung enthält, wird in eine Mikrotiterplatte gegeben und in den Verhältnissen 1000:1 bis 1:1000 mit einer wäßrigen Lösung von 0.1-1000 mM Umbelliferon-Natriumsalz gemischt und bei 0°C bis 25°C, vorzugsweise bei 0-10°C, besonders bevorzugt bei 4°C inkubiert. Die Lösung, die das Protein enthält, besteht vorzugsweise aus einem Phosphatpuffer, beispielsweise mit pH 7.0, der neben dem Protein zusätzlich 1-500 mM Umbelliferon-Natriumsalz oder dessen Derivate enthält. Die Umbelliferonkonzentration ist jedoch immer geringer als die der Umbelliferon-Kristallisierungslösung.

Über einen Zeitraum von drei Monaten bilden sich Kristalle, die unterschiedlich aussehen können.

### Beispiel 2

### Kristallisierung von Glycoproteinen

Eine Glycoproteinlösung, die 1 mg Glycoprotein pro ml Lösung enthält, wird in eine Mikrotiterplatte gegeben und im Verhältnis 1000:1 bis 1:1000 mit einer wäßrigen Lösung von 50 mM 4-Methyl-Umbelliferon-Natriumsalz gemischt und bei 4°C für 1 Tag inkubiert. Die entstehenden Kristalle werden so lange gezüchtet, bis möglichst viele glatte Oberflächen vorhanden sind. Dies wird mikroskopisch beurteilt. Wenn das Ergebnis zufriedenstellend ist, können die Kristalle untersucht werden.

### Beispiel 3

### Kristallisierung von Nucleinsäuren

Eine Lösung, die etwa 300 µg Nucleinsäure pro ml Lösung enthält, wird in eine Mikrotiterplatte gegeben und im Verhältnis 1000:1 bis 1:1000 mit einer wäßrigen Lösung von 50 mM 4-Methyl-Umbelliferon-Natriumsalz gemischt und bei 4°C inkubiert. Über einen Zeitraum von drei Monaten bilden sich Kristalle, die unterschiedlich aussehen können. Die entstehenden Kristalle werden so lange gezüchtet, bis möglichst viele glatte Oberflächen vorhanden sind. Dies wird mikroskopisch beurteilt.

## Patentansprüche

1. Verfahren zur Kristallisierung von Makromolekülen, dadurch gekennzeichnet, daß die Kristallisierung in Gegenwart eines oder mehrerer 4-Alkyl-Umbelliferon-Salze erfolgt, wobei die Alkylgruppe geradkettig oder verzweigt sein kann und 1 bis 18 Kohlenstoffatome aufweist.

2. Verfahren nach Anspruch 1, dadurch gekennzeichnet, daß die Alkylgruppe 1 bis 8 Kohlenstoffatome aufweist.

3. Verfahren nach Anspruch 1, dadurch gekennzeichnet, daß die Alkylgruppe 1 bis 4 Kohlenstoffatome aufweist.

4. Verfahren nach Anspruch 1, 2 oder 3, dadurch gekennzeichnet, daß das Salz ein Alkali- und/oder Erdalkalisalz ist.

5. Verfahren nach einem der vorhergehenden Ansprüche, dadurch gekennzeichnet, daß das 4-Alkyl-Umbelliferon-Salz ein 4-Methyl-Umbelliferon-Salz ist.

6. Verfahren nach Anspruch 5, dadurch gekennzeichnet, daß das 4-Methyl-Umbelliferon-Salz das Natriumsalz ist.

7. Verfahren nach einem dervorhergehenden Ansprüche, dadurch gekennzeichnet, daß die Kristallisierung aus wäßriger Lösung erfolgt.

8. Verfahren nach einem der vorhergehenden Ansprüche, dadurch gekennzeichnet, daß die, Makromoleküle ausgewählt sind aus Polypeptiden, Proteinen, Glycoproteinen und Nukleinsäuren.

9. Verfahren nach einem der vorhergehenden Ansprüche, dadurch gekennzeichnet, daß eine Lösung des Makromoleküls mit einer Konzentration von 0,1 bis 10.000 µg/ml im Verhältnis 1000:1 bis 1:1000 mit einer, 0,1 bis 1000 mM 4-Methyl-Umbelliferon-Natriumsalz-Kristallisierungslösung gemischt und bei 0°C bis 25°C inkubiert wird.

10. Verfahren nach Anspruch 9, dadurch gekennzeichnet, daß die Makromoleküllösung eine Proteinlösung in einem Phosphatpuffer bei einem pH von 3,0-9,0 ist und die Lösung zusätzlich 1-500 mM 4-Methyl-Umbelliferon-Natriumsalz enthält, wobei die 4-Methyl-Umbelliferon-Natriumsalz-Konzentration in dieser Lösung geringer ist als die 4-Methyl-Umbelliferon-Natriumsalz-Konzentration in der Kristallisierungslösung.

11. Kristallisiertes Makromolekül, dadurch gekennzeichnet, daß es nach einem Verfahren gemäß einem der Ansprüche 1-10 herstellbar ist.

12. Kristallisiertes Makromolekül nach Anspruch 11, dadurch gekennzeichnet, daß es 4-Methyl-Umbelliferon in der Form des Natriumsalzes aufweist.

13. Pharmazeutische Zusammensetzung, dadurch gekennzeichnet, daß sie als Wirkstoff eine pharmakologisch wirksame Menge eines gemäß einem der Ansprüche 1-10 kristallisierten Makromoleküls und gegebenenfalls einen oder mehrere herkömmliche, pharmazeutisch akzeptable Träger umfaßt.
